# EUROPEAN PATENT SPECIFICATION

(11) **EP 0 758 912 B1**
(45) Date of publication and mention of the grant of the patent: **27.11.2002**
(21) Application number: 95919719.5
(22) Date of filing: 12.05.1995
(51) Int. Cl.: A61M 16/20, F16K 31/06

(54) **REGULATOR VALVE**
REGELUNGSVENTIL
VALVE DE REGULATEUR

(30) Priority: 13.05.1994 SE 9401661
(43) Date of publication of application: 26.02.1997
(73) Proprietor: ENGSTRÖM MEDICAL AB, S-161 02 Bromma (SE)
(72) Inventor: BRÖMSTER, Leif, S-171 57 Solna (SE)
(74) Representative: Hyltner, Jan-Olof
(86) International application number: SE9500533
(87) International publication number: WO95031241

(56) References cited:
- DE-A- 3 725 590
- US-A- 4 643 394
- US-A- 4 848 726
- US-A- 4 921 210
- PATENT ABSTRACTS OF JAPAN, Vol. 8, No. 182, M-319; & JP,A,59 073 680 (MATSUSHITA DENKI SANGYO K.K.), 25 April 1984.

## Description

### FIELD OF THE INVENTION

The present invention relates to a regulator valve for proportional control of the flow of fluids, such as gases, for example air, oxygen, nitrogen etc.

The valve is particularly intended for use in an apparatus for supplying and regulating of inhalation gas to a patient in hospital care, in particular an anaesthesia device or intensive care respirator, as described in US-A-4 702 242.

### STATE OF THE ART

Valves for control of gas flows are already known in many different forms. One known principal is to allow the gas to pass through a gap as a radial flow. In such a valve the flow is substantially proportional to the gap height at constant pressure differential.

Such valves are normally regulated in that the gap height is determined by a disc which is positioned on a spindle directly opposite an annular valve seat. Axial displacement of the spindle changes the gap height. Displacement normally occurs by means of a solenoid, but other types of manoeuvring means also exist.

Such prior art regulator valves however have many disadvantages. For example the regulation range of the valve is often far too small, at least the range where the valve has proportional, linear characteristics.

Additionally the valve often has hysteresis due to the magnetic material in the solenoid. Such hysteresis has the effect that the characteristic curve is different depending on whether the valve is being opened or closed.

The aforementioned control spindles normally slide in some type of guide or bearing, as a result of which frictional forces occur, which also results in hysteresis. Additionally the frictional forces cause the valve to move in a stepwise manner during adjustment or to move in an uncontrolled manner, to a certain extent as the frictional forces are overcome. US-A-4 643 394 and JP-A-59-73680 describe regulator valves of the type where the valve element is guided such that frictional forces ensue.

### SUMMARY OF THE INVENTION

The object of the present invention is to arrive at an arrangement for the regulation of such a gap valve in order to achieve a regulator valve having a substantially linear relationship between flow and a control magnitude which is preferably an electric current.

A further object of the invention is to arrive at a regulator valve which is very precise and gives repeatable values for the regulation of the flow.

A still further object of the invention, in as far as possible, is to eliminate the frictional forces and the magnetic hysteresis which prior art solenoid valves possess.

In order to achieve these objects a regulator valve according to the invention has the features which are defined in the appended claims.

Because no frictional forces ensue in the regulator valve according to the invention, the only moving part in the valve, the former and the parts which are connected to the same, is light weight. In this way fast response time and good repeatability without uncontrolled overshooting, which depend upon the moving mass, can be achieved during flow adjustment. Because the moving part is freely suspended there is no friction which can interfere with the flow adjustment.

Magnet systems of this type have linear charactristics and no hysteresis. The equilibrium of forces in the valve is decided only by the incoming pressure and the helical spring in the valve.

Helical springs, in contrast to leaf springs, normally have linear characteristics. Conventional linear valves available on the market are built up as a solenoid in which the moving part is made of iron. The solenoid has non-linear characteristics which means that the spring in such a valve must be made non-linear if one wishes to obtain a linear valve (flow proportional to input power). In order to achieve this, the spring in such a valve must be a specially shaped leaf spring.

### BRIEF DESCRIPTION OF THE DRAWINGS

The invention will now be described in more detail with reference to preferred embodiments of the invention and with reference to the accompanying drawings.

Fig. 1 is a cross-sectional view of a preferred embodiment of the regulator valve according to the present invention.

Fig. 2 is a cross-sectional view of the regulator valve according to Fig. 1, however in a second operational position.

Fig. 3 is a plan view of a spring washer which is used in the regulator valve according to Fig. 1.

Fig. 4 is a diagram which shows the relationship between the flow and the electrical regulating magnitude, which in this case is a current strength.

Fig. 5 is a plan view similar to Fig. 3 of an alternative embodiment of the spring washer.

Fig. 6 is an enlarged component cross-section of the valve seat and the valve element in the regulator valve according to Fig. 1 and 2.

Fig. 7 is a cross-section corresponding to Fig. 6 of an alternative embodiment of a valve seat and valve element.

### DETAILED DESCRIPTION OF THE INVENTION

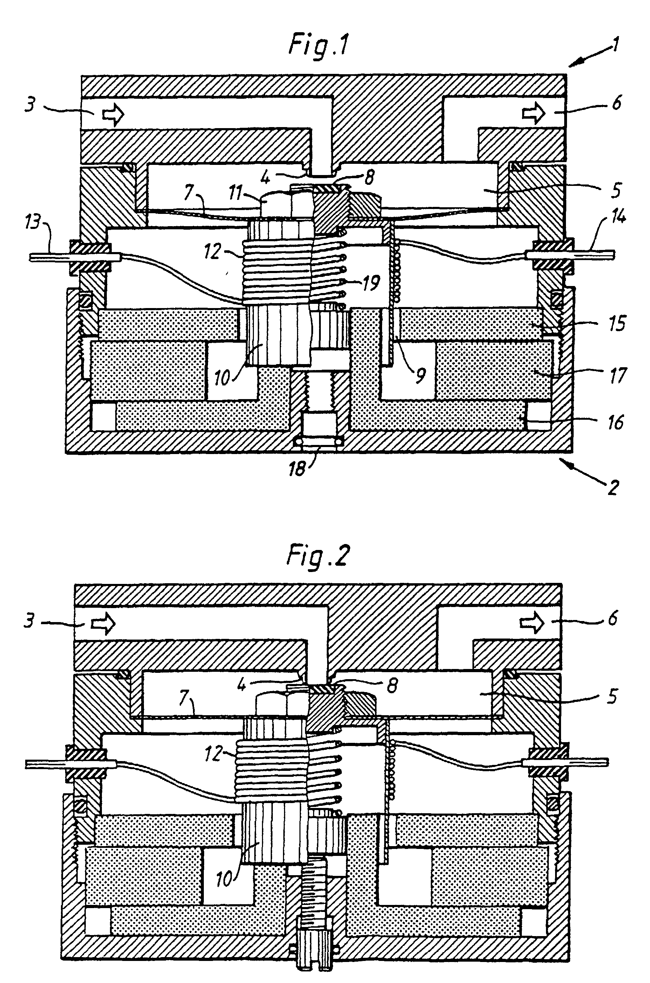

Fig. 1 shows a preferred embodiment of the regulator valve according to the invention in cross-section.

The regulator valve comprises a valve housing 1 and a regulator unit 2. The valve housing comprises an inlet channel 3 for the incoming flow which ends in a valve seat 4. The valve seat 4 has an orifice arranged centrally in a cylindrical valve chamber 5 which is provided on one side with an outlet 6.

The valve chamber 5 is delimited downwardly in Fig. 1 by a preferably circular membrane 7. In the centre of the membrane there is planar rubber plate 8 which is positioned directly in front of the aforesaid valve seat 4 against which it can form a seal. The membrane is fixedly clamped at its periphery between the valve housing 1 and the regulator unit 2.

On the other side of the membrane 7, directly in front of the rubber plate, a tube-shaped former 10 is arranged, said former being fixed to the membrane 7 by means of a nut 11, preferably of aluminium. On the former there is a coil 12 of electrically conducting wire. The ends of the coil 12 are connected to terminals 13, 14 which pass through the walls of the regulator unit 2.

The former is positioned in an annular air gap 9 formed by two magnetic yokes 15, 16, whereby a radial magnetic field is maintained in the annular air gap 9 by means of a permanent magnet 17 (of ring-type) positioned between the magnetic yokes 15, 16. The height of the air gap 9 is defined by the first yoke 15 which is ring-shaped. The coil 12 and the former 10 are free to move in the air gap 9 without touching the yokes 15, 16.

The regulator unit 2 is provided with arrangements for securely holding the yokes 15, 16 and the permanent magnet 17 in a particular position so that the annular air gap 9 is well defined and as rotationally symmetric as possible. There is also a central hole 18, which is provided with a screw (not shown in Fig. 1). A helical spring 19 is arranged between the screw and the above-lying membrane, i.e. axially within the coil.

It can thus be seen that the regulator housing described above is very much like the inside of a loud speaker and, in a practical embodiment of the regulator valve, a disassembled loud speaker is also used, where the loud speaker membrane is replaced by the aforementioned membrane.

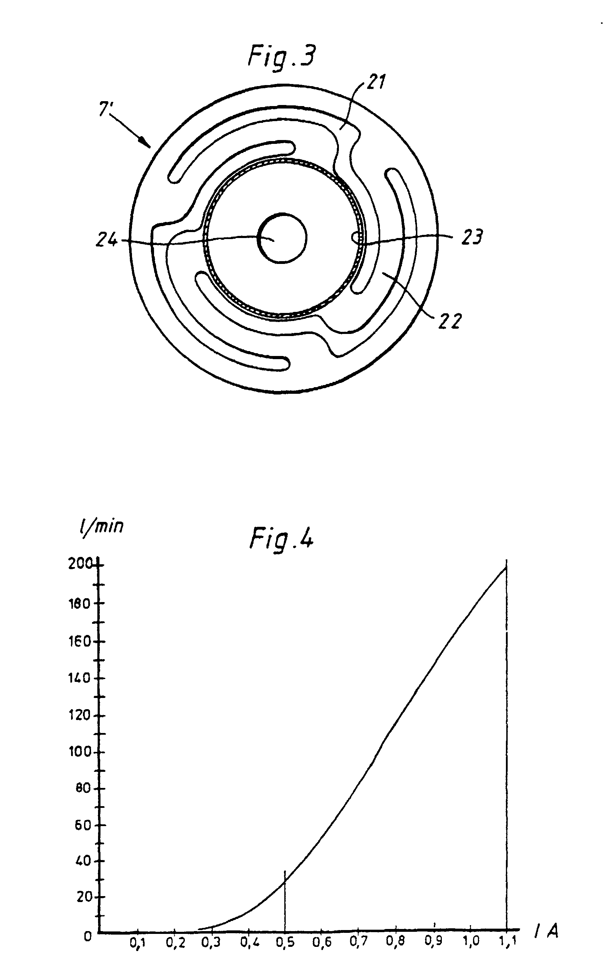

The membrane 7 is shown in more detail in Fig. 3. The membrane consists of a circular steel washer 7' provided with several recesses 21 as shown in the figure. The recesses together form three partially ring-shaped sprung legs 22 which are as long as possible. In this way the spring force is not too large, which is desirable. The former 10 is attached to the spring washer 7', as shown with the dashed lines 23 in Fig. 3. In the middle of the spring washer there is a hole 24 intended for application of a screw and nut.

In this way a spring washer is obtained where the axial deflection of the centre of the washer with respect to its periphery is essentially proportional to the deflection force, naturally within certain limits. The spring washer also resists the tendency of the former to assume an oblique position, so that this always remains substantially axially aligned.

From the above description the function of the regulator valve ought to be clear. A current is supplied to the terminals 13 and 14 and passes through the coil 12. The current cooperates with the radial magnetic field produced by the permanent magnet 17 and the yokes 15 and 16 in the air gap 9 therebetween. A force therefore rises which tends to displace the coil axially in one of the two directions depending on the direction of the supplied current, whereby the force is directly proportional to the current.

The coil is pre-loaded upwardly in Fig. 1 by means of the helical spring 19 and the membrane 7 or the spring washer 7'. The rubber plate 8 is thereby pressed against the valve seat 4 with a predetermined force and the inlet is shut. As the supplied current increases the force produced by the current and the magnetic field will oppose and cancel the sum of the aforesaid spring forces and the pressure which the inlet pressure exerts on the rubber plate, whereby the valve seat opens and a flow is obtained from the inlet 3 to the valve chamber 5 and the outlet 6.

When the current increases further, the coil and the membrane will be displaced further downwards to a new stable position determined by the opposing spring forces. Due to this the distance between the rubber plate 8 and the valve seat 4 will also increase. The gas flow past the valve seat is substantially proportional to the distance between the rubber plate and the valve seat.

The relationship between the gas flow and the current in the coil is clearer from Fig. 4. As is clear from the curve, the flow is almost zero up until a current strength of about 0.3 A, where the spring forces are essentially opposed. From about 0.5 A and up until just over 1.1 A the gas flow is almost entirely proportional to the current strength, said range corresponding to a gas flow between about 30-200 litres per minute at an inlet pressure of 294 kPa (3 kp/cm²) and an outlet pressure of about 9.8 kPa (0.1 kp/cm²) for the embodiment forming the basis for Fig. 4.

The linearity of the relationship between the gas flow and current strength can be improved or influenced by the exact design of the valve seat and the rubber plate. The linearity of the springs' characteristics are also influ-enced.

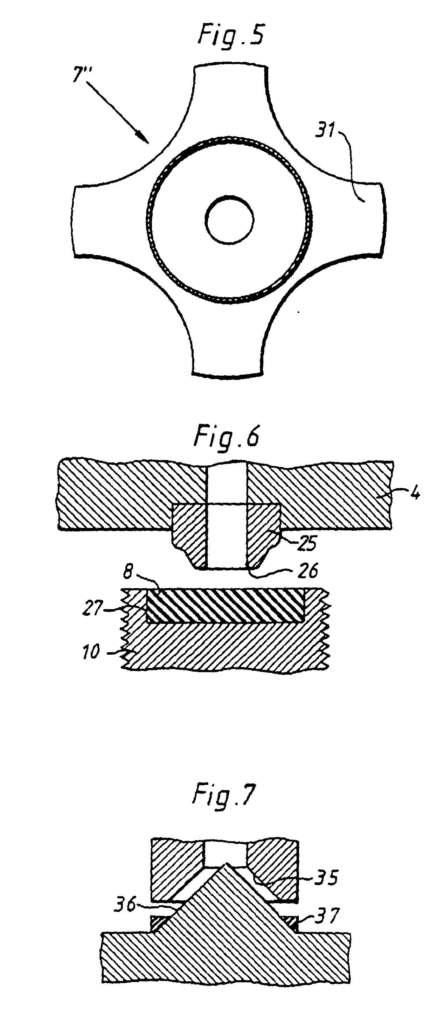

Fig. 6 shows an enlarged view of the valve seat 4 and the valve element 8. The valve seat consists of a nipple 25 which extends outwardly from the surface of the valve chamber 5. The nipple 25 has a surface-ground lower edge 26. The valve element consists of said nut 11 which has a countersink 27 in which the rubber plate 8 is arranged. The distance between the plate 8 and the lower edge 26 forms the gap through which the gas flow passes.

The coil consists of a few turns, preferably 60-80 turns, of conductive copper wire which can have a square cross-section in order to make better use of the space in the air gap 9. Other known characteristics and features from loud speaker technology can also be applied to in the present invention.

The former in the shown regulator valve is completely without iron core, which leads to a very light and thereby very quick construction. Since there is no iron core no remanence or hysteresis occurs which can affect the precision and the repeatability of the regulation.

The former is supported only by the membrane and is displaced in the air gap without contacting the yokes. Thus no friction occurs for the former, which thus makes it very easily moveable. A very precise and repeatable regulation can be achieved according to the invention.

It is noted that one or more of the helical spring 19 and the screw in the hole 18 as well as the nut 11 will suitably be manufactured from plastics material or aluminium in order to be inert from a magnetic point of view and as light as possible.

The membrane 7 or the spring washer 7' can be made of steel so that they have good spring characteristics.

It will be seen that the air in the valve chamber 5 can pass through the hole in the spring washer 7' down into the space under the membrane. The gas pressure in the valve chamber 5 therefore does not influence the membrane or subject the coil to any force other than in the closed position, where the inlet pressure acts on the surface of the rubber plate. The regulator unit is sealed in such a way that the gas can not leak out to the surroundings.

The spring washer 7' can alternatively be provided with a sealing membrane so that the gas is locked in above the membrane. In this way the pressure at the outlet will act on the membrane and a variation of this pressure will influence the gap height. Since however the pressure at the outlet is very close to atmospheric pressure and moreover substantially constant, this influence will not result in any practical inconvenience in certain applications. The membrane has the function of keeping the former aligned with the axis of symmetry of the air gap. The necessary spring force is achieved by said helical spring.

Fig. 5 shows an alternative embodiment of the spring washer which forms the membrane 7. This spring washer 7'' has four radial legs 31 which can give the desired spring effect in an axial direction and give stability in a sideways direction. It is also clear that additional variations on the spring washer are available to a skilled man.

It is also clear that the yokes 15, 16 have to be manufactured from ferro-magnetic material which does not give rise to any risk of saturation due to the magnetic field in the coil.

The permanent magnet can of course be replaced by an electromagnet if so desired. In this way a quadratic relationship between the current and force can be obtained, i.e. between the current and the distance between the valve plate and the valve seat. The permanent magnet can be of a conventional construction or can be manufactured from highly permanent magnetic material such as cobalt-samarium etc.

As an alternative the valve seat 4 can be formed as an O-ring seal and the rubber plate 8 can be replaced by a hard plate, of for example hard plastics. The former can be cast in a mould where the mid-portion is raised in order to form the plate. The former can also have integrated therein the spring washer or membrane 7, whereby a regulator valve with few parts is obtained.

It is possible to form the valve seat and the valve element as conical co-operating elements, whereby the same function is obtained, but with a larger stroke-length for the coil. Such an embodiment is shown in more detail in Fig. 7. The valve seat is thereby formed with an inner conical portion 35 and the valve element has a complementary formed conical portion 36. In order to achieve sure sealing in the closed position, the valve element can be provided with a rubber seal 37 at the foot of the conically formed portion. In this embodiment it is of importance that the conically shaped portion is exactly rotationally symmetrical and not skew, which influences the characteristics of the regulator valve.

The invention has been described above with reference to preferred embodiments thereof. It is however intended that the invention can be modified in many respects by a skilled man reading this description and it is intended that such modifications which are obvious for the skilled man are to be included in the framework of the invention. The invention is only limited by the appended claims.

## Claims

1. Regulator valve for control of fluid, for example an inhalation gas flow in an anaesthesia device or an intensive care respirator, comprising
an inlet (3) for the flow;
a valve seat (4), which is connected with the inlet (3);
a valve chamber (5), which is connected with the valve seat (4);
an outlet (6) for the flow, which is connected with the valve chamber (5);
a valve element (8), which is arranged to co-operate with the valve seat (4) and to regulate the flow through the regulator valve; as well as
a regulator unit (2), which is connected with the valve element (8) in order to regulate the distance between the valve seat (4) and the valve element (8),
a yoke arrangement (15, 16), which forms an annular air gap (9) and a magnet (17) arranged in connection with the yoke arrangement in order to produce a magnetic field in the air gap (9) ;
a coil arrangement (10, 12), whereby the valve element (8) is arranged on one end of the coil arrangement (10);
a membrane (7), which is arranged to support the coil arrangement (10 and/or 12) in a position such that the valve element (8) is positioned directly in front of the valve seat (4) and the coil arrangement (10 and/or 12) is positioned in, and is moveable in, the air gap (9);
that the valve element (8) and the coil arrangement (10, 12) form a unit suspended on the membrane (7);
**characterized in**
**that** a spring arrangement (19, 7) is formed by a helical spring (19) arranged centrally within the coil arrangement (10, 12) and which is arranged to pre-load the valve element (8) towards or away from the valve seat (4), that the membrane is formed by a spring washer (7', 7") with essentially linear spring characteristics, that the coil arrangement comprises a former (10) fixed on the membrane (7), and that the membrane (7) is formed by a guide for the former to keep the coil arrangement (10, 12) concentric with the air gap (9), so that the coil arrangement (10, 12) is movable in the air gap (9) without touching the yoke arrangement (15, 16).

2. Regulator valve according to claim 1, **characterized by** an adjustment screw, which, is arranged to act on one end of the helical spring (19) for adjustment of the contact pressure between the valve plate (8) and the valve seat (4) in the closed position of the regulator valve.

3. Regulator valve according to any one of the preceding claims, **characterized in that** the valve element (8) consists of a nut (11) attached to the membrane (7) and the former (10) as well as a rubber plate (8) which is countersunk in a recess (27) in said nut (11).

4. Regulator valve according to claim 3, **characterized in that** the valve seat consists of a nipple (25) which extends outwardly from the surface of the valve chamber (5) and is provided with a planar outer edge (26).

5. Regulator valve according to any one of the preceding claims, **characterized in that** the valve element (8) consists of a comically formed portion (36) and **in that** the valve seat (4) consists of a complementary formed portion (35).

## Patentansprüche

1. Steuer- bzw. Regelventil zum Steuern bzw. Regeln eines Fluids, z.B. eines Inhalationsgasstroms in einer Anästhesie- bzw. Narkosevorrichtung oder einem Intensivpflegerespirator bzw. -beatmungsgerät, aufweisend:
einen Einlass (3) für den Strom;
einen Ventilsitz (4), der mit dem Einlass (3) verbunden ist;
eine Ventilkammer (5), die mit dem Ventilsitz (4) verbunden ist;
einen Auslass (6) für den Strom, der mit der Ventilkammer (5) verbunden ist;
ein Ventilelement (8), das so angeordnet ist, um mit dem Ventilsitz (4) zusammenzuarbeiten und um den Strom durch das Steuer- bzw. Regelventil zu steuern bzw. zu regeln; ferner
eine Steuer- bzw. Regeleinheit (2), die mit dem Ventilelement (8) verbunden ist, um den Abstand zwischen dem Ventilsitz (4) und dem Ventilelement (8) zu steuern bzw. zu regeln,
eine Jochanordnung (15, 16), die einen ringförmigen Luftspalt (9) bildet, und einen Magnet (17), der in Verbindung mit der Jochanordnung angeordnet ist, um ein Magnetfeld in dem Luftspalt (9) zu erzeugen;
eine Spulenanordnung (10, 12), wobei das Ventilelement (8) an einem Ende der Spulenanordnung (10) angeordnet ist;
eine Membran (7), die so angeordnet ist, um die Spulenanordnung (10 und/oder 12) in einer Position derart zu stützen bzw. zu tragen, dass das Ventilelement (8) unmittelbar vor dem Ventilsitz (4) positioniert ist und die Spulenanordnung (10 und/oder 12) in dem Luftspalt (9) positioniert und bewegbar ist;
dass das Ventilelement (8) und die Spulenanordnung (10, 12) eine Einheit bilden, die an der Membran (7) aufgehängt ist;
**dadurch gekennzeichnet, dass**
eine Federanordnung (19, 7) durch eine Schraubenfeder (19) gebildet ist, die zentral bzw. mittig innerhalb der Spulenanordnung (10, 12) angeordnet ist, und so angeordnet ist, um das Ventilelement (8) in Richtung zu dem Ventilsitz (4) oder von dem Ventilsitz (4) weg vorzuspannen, dass die Membran durch eine Federscheibe bzw. einen Federring (7', 7") mit im wesentlichen linearen Federcharakteristika bzw. Federkennlinie gebildet ist, dass die Spulenanordnung einen Spulenkörper (10) aufweist, der an der Membran (7) befestigt ist, und dass die Membran (7) durch eine Führung für den Spulenkörper gebildet ist, um die Spulenanordnung (10, 12) konzentrisch mit dem Luftspalt (9) zu halten, so dass die Spulenanordnung (10, 12) in dem Luftspalt (9) bewegbar ist, ohne die Jochanordnung (15, 16) zu berühren.

2. Steuer- bzw. Regelventil nach Anspruch 1, **gekennzeichnet durch** eine Einstellschraube, die so angeordnet ist, um auf ein Ende der Schraubenfeder (19) zum Einstellen des Kontaktdruckes zwischen der Ventilplatte (8) und dem Ventilsitz (4) in der geschlossenen Position des Steuer- bzw. Regelventils einzuwirken.

3. Steuer- bzw. Regelventil nach einem der vorhergehenden Ansprüche, **dadurch gekennzeichnet, dass** das Ventilelement (8) sowohl aus einer Mutter (11), die an der Membran (7) und dem Spulenkörper (10) angebracht ist, als auch aus einer Gummiplatte (8) besteht, die in einer Ausnehmung (27) in der Mutter (11) versenkt ist.

4. Steuer- bzw. Regelventil nach Anspruch 3, **dadurch gekennzeichnet, dass** der Ventilsitz aus einem Nippel (25) besteht, der sich von der Fläche der Ventilkammer (5) auswärts erstreckt und mit einem ebenen, äußeren Rand (26) versehen ist.

5. Steuer- bzw. Regelventil nach einem der vorhergehenden Ansprüche, **dadurch gekennzeichnet, dass** das Ventilelement (8) aus einem kegelförmig bzw. konisch geformten Abschnitt bzw. Bereich (36) besteht und dass der Ventilsitz (4) aus einem komplementär geformten Abschnitt bzw. Bereich (35) besteht.

## Revendications

1. Valve de régulateur pour contrôle de fluide, par exemple un flux de gaz d'inhalation dans un dispositif d'anesthésie ou un respirateur pour soins intensifs, comprenant
une entrée (3) pour le flux ;
un siège de valve (4), qui est relié à l'entrée (3) ;
une chambre de valve (5), qui est reliée au siège de valve (4) ;
une sortie (6) pour le flux, qui est reliée à la chambre de valve (5) ;
un élément de valve (8), qui est agencé pour coopérer avec le siège de valve (4) et pour réguler le débit au travers de la valve de régulateur ; ainsi
qu'une unité de régulateur (2), qui est reliée à l'élément de valve (8) de façon à réguler la distance entre le siège de valve (4) et l'élément de valve (8) ;
qu'un ensemble d'étrier (15, 16) qui forme un entrefer annulaire (9) et un aimant (17) agencé en liaison avec l'ensemble d'étrier de façon à produire un champ magnétique dans l'entrefer (9) ;
qu'un ensemble de bobinage (10, 12), l'élément de valve (8) étant agencé à une extrémité de l'ensemble de bobinage (10) ;
qu'une membrane (7), qui est agencée pour supporter l'ensemble de bobinage (10 et/ou 12) dans une position telle que l'élément de valve (8) est placé directement en regard du siège de valve (4) et l'ensemble de bobinage (10 et/ou 12) est placé dans, et est mobile dans, l'entrefer (9) ;
l'élément de valve (8) et l'ensemble de bobinage (10 et/ou 12) formant une unité suspendue sur la membrane (7) ;
**caractérisée en ce**
**qu'**un ensemble de ressort (19, 7) est formé par un ressort hélicoïdal (19) agencé de façon centrale dans l'ensemble de bobinage (10 et/ou 12) et qui est agencé pour pré-contraindre l'élément de valve (8) vers le siège de valve (4) ou le pré-contraindre pour l'en écarter,
**que** la membrane est formée par une rondelle de ressort (7', 7'') possédant des caractéristiques élastiques essentiellement linéaires,
**que** l'ensemble de bobinage comprend un gabarit (10) fixé sur la membrane (7), et
**que** la membrane (7) est formée par un guide pour le gabarit destinée à maintenir l'ensemble de bobinage (10, 12) concentrique avec l'entrefer (9), de sorte que l'ensemble de bobinage (10, 12) soit mobile dans l'entrefer (9) sans contact avec l'ensemble d'étrier (15, 16).

2. Valve de régulateur suivant la revendication 1, **caractérisée par** une vis de réglage, qui est agencée pour agir sur une extrémité du ressort hélicoïdal (19) pour régler la pression de contact entre la plaque de valve (8) et le siège de valve (4) dans la position fermée de la valve de régulateur.

3. Valve de régulateur suivant l'une quelconque des revendications précédentes, **caractérisée en ce que** l'élément de valve (8) consiste en un écrou (11) fixé à la membrane (7) et au gabarit (10) ainsi qu'à une plaquette de caoutchouc (8) qui est enchâssée dans un évidement (27) dans ledit écrou (11).

4. Valve de régulateur suivant la revendication 3, **caractérisée en ce que** le siège de valve consiste en un manchon (25) qui s'étend vers l'extérieur à partir de la surface de la chambre de valve (5), et est pourvu d'un bord extérieur plan (26).

5. Valve de régulateur suivant l'une quelconque des revendications précédentes, **caractérisée en ce que** l'élément de valve (8) consiste en une partie (36) de forme conique et **en ce que** le siège de valve (4) consiste en une partie (35) de forme complémentaire.
